(19) Europäisches Patentamt / European Patent Office / Office européen des brevets

(11) **EP 4 417 183 B1**

(12) **EUROPEAN PATENT SPECIFICATION**

(45) Date of publication and mention
of the grant of the patent:
**28.01.2026 Bulletin 2026/05**

(21) Application number: **24158569.4**

(22) Date of filing: **20.02.2024**

(51) International Patent Classification (IPC):
**A61K 8/22** (2006.01)     **A61Q 5/00** (2006.01)

(52) Cooperative Patent Classification (CPC):
**A61K 8/22; A61Q 5/00**

(54) **COSMETIC METHOD FOR HAIR WITH OZONE**

KOSMETISCHE METHODE FÜR HAARE MIT OZON

MÉTHODE COSMÉTIQUE POUR CHEVEUX À L'OZONE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **20.02.2023 IT 202300002838**

(43) Date of publication of application:
**21.08.2024 Bulletin 2024/34**

(73) Proprietor: **Purò S.r.l.**
**40050 Argelato BO (IT)**

(72) Inventors:
• **Montanari, Federico**
**40050 Argelato BO (IT)**
• **Venturi, Flavio**
**40050 Argelato BO (IT)**
• **Garoia, Flavio**
**40050 Argelato BO (IT)**
• **Marchesini, Davide**
**40050 Argelato BO (IT)**

(74) Representative: **Perani & Partners S.p.A.**
**Corso Europa, 15**
**20122 Milano (IT)**

(56) References cited:
**WO-A1-2023/037208     GB-A- 2 313 779
GB-A- 820 463**

**Description**

**FIELD OF THE INVENTION**

The field of the present invention relates to a cosmetic method for hair care based on ozone.

**STATE OF THE ART**

Ozone therapy is an ozone-based therapy or treatment that is known to produce a number of benefits able to combat a wide range of pathologies (herniated discs, acne, phlebitis, fibromyalgia, infections, haemorrhoids, arthritis, cellulite, ulcers, burns, osteoporosis). This treatment significantly improves the body's defence systems against the action of free radicals, which are mainly responsible for cellular ageing and tissue oxidation.

Ozone $O_3$ is a soluble gas in aqueous solution (~13 times more than $O_2$ at 0-30°C) with a solubility inversely proportional to temperature and pH. It is also characterised by a high oxidative potential (redox potential of +2.07 V *vs.* redox potential of the oxygen molecule $O_2$ of +1.23 V), which is lower than only some substances, and significantly higher than that of chlorine (+1.36 V).

It forms naturally in the human body as it is produced by white blood cells called neutrophils; thus, within the body, it performs the delicate task of counteracting the action of bacteria and fungi and, in general, all abnormal elements in the body.

In addition to its therapeutic use, ozone can also be used in cosmetics, for example for hair care.

At the state of the art, ozone is used during technical hair treatments, such as colouring, bleaching or styling, more specifically during the application period of the technical treatment or in progression thereof. Such use usually leads to advantages in terms of the duration of the colour or the blow-dry, and the intensity of hair colouring. In other words, ozone has an adjuvant or enhancing effect on the performance of the technical treatment carried out on a user's hair.

In particular, several earlier documents show that the use of ozone is functional in terms of the *performance* of technical treatment, *e.g. in* order to speed up the process of technical treatment, to make it more efficient or characterised by better results (e.g. a greater bleaching effect or a more vivid and intense hair colour).

Examples of such applications are described in the following patent documents:
FR3010305 describes a perming method comprising the following steps: rolling the hair in curlers and applying a "first perming agent" having a reducing action, in order to dilate and soften the hair; rinsing the hair to remove the first perming agent; applying to the hair an "intermediate treating/conditioning agent" (or "intermediate agent" having a "neutralising" capacity), i.e., "one or more compounds selected from the group consisting of: ascorbic acid, an ascorbic acid derivative and a salt of ascorbic acid' and ozone, in order to oxidise the intermediate agent and reconstruct the disulphide bridges; apply a "second permanent agent" to the hair, when the hair is dry, to further reconstruct the disulphide bridges, in a time of between 1 and 3 minutes. In accordance with the method described in FR3010305, ozone has an oxidising function and, in this sense, together with the intermediate agent, acts as a "neutralising agent", i.e. capable of recomposing the disulphide bonds in the hair, following the use of the first permanent agent with the opposite action, i.e. reducing, stabilising the new shape (wave) of the hair. In addition, the application of ozone takes place during the technical treatment of the perm. The authors of FR3010305 also consider that, in a humid environment, the reconstruction of the disulphide bridges proceeds in a direction that weakens the hair shape obtained during the drying phase and, when the hair dries, the wave shape is not the same as when the hair was wet. The ozone concentration according to the method of FR3010305 is comprised between 0.5 and 1 ppm; the reason why the ozone concentration is set to a maximum value of 1 ppm is related to safety considerations with respect to ozone sprays, and the human body. As described in FR3010305, the treatment duration is comprised between 3 and 15 minutes.

EP0103547 describes an apparatus for generating steam and ozone - ozonised steam - in controllable quantities for the treatment of both skin and hair. For hair treatment, a hair dryer (or bell) is used, especially when applying dyes or perms. Treatment with the steam/ozone mixture described in EP0103547 can enhance the bleaching process of hair keratins, and improve hair dyeing and pigmentation processes. According to the authors of EP0103547, ozone prevents itching and improves the action of keratin, helping, in combination with heat, the evaporation of ammonia contained in hair dyes and bleaching products.

GB820463 concerns a method for the improvement of hair dyeing, bleaching and/or styling processes. A steam and ozone generating apparatus is used in combination with hair treatments. The invention described in GB820463 accelerates and improves the oxidation of dyes used in hair dyeing; improves the action of lightening/bleaching products; and is useful in the neutralisation of perms.

*Problem of known technique*

As already mentioned, well-known ozone-based cosmetic methods are intended to enhance the technical treatment by virtue of the oxidising power of $O_3$.

Ozone, however, precisely because of its chemical properties, is harmful to the hair if used improperly: it can lead to more or less reversible structural and functional damage to the hair, more specifically damage to the hair's protein components.

Therefore, there is a need to develop new techniques for using ozone during hair treatments, minimising the harmful effects of the molecule while exploiting its beneficial and functional properties.

## SUMMARY OF THE INVENTION

The Applicant has identified a method for cosmetic hair treatment using ozone. In particular, the cosmetic hair care method comprises the following steps:

a) conduct a technical hair treatment, comprising at least the sub-phases of

a1) apply to the hair one or more compositions functional to the technical treatment,
a2) leave one or more compositions functional to the technical treatment on the hair,
(a3) washing the hair to remove one or more compositions functional to the technical treatment,

b) apply ozone to damp hair

for a period of time > 0.5 and < 21 minutes,
at an ozone dosage ranging between $\geq 0.01$ and $\leq 2000$ mg/h, excluding the ozone dosage ranging between 180 and 400 mg/h, wherein the phase of applying ozone to damp hair (b) takes place at the end of the phase of conducting a technical treatment on the hair (a) the technical treatment is selected from: hair reconstruction, hair conditioning, colouring, bleaching, perming, and combinations of the foregoing.

*Advantages of the invention*

The cosmetic method of the invention is not intended to accentuate or improve the result or *performance of* the technical treatment applied to the hair, but rather focuses on the partial or total regeneration of the primordial structure of the hair pre-treatment technique, i.e. by acting at the level of the disulphide bridges, preferably cysteine disulphide bridges of the hair. This is achieved by the use of ozone following technical parameters described in the set of claims and in the following description.

The present invention differs from common ozone-based cosmetic methods in that:

- involves applying ozone to the hair at a specific dosage of ozone and within a specific time frame. Higher dosages or timescales of ozone application could compromise the constitution of the hair, e.g. by damaging or deteriorating it. From the examples given, it will be more evident that the use of $O_3$ over a *prolonged* period of time produces structural, and thus also functional, damage to the hair;
- is versatile since it can be used for hair care following various technical treatments; moreover, versatility of use must also be understood from the point of view of the technical instrumentation available in beauty salons. In fact, the phase of applying ozone to the hair (b) can be done by means of different types of professional instruments such as, for example, a hair brush, a hair comb, a hair dryer (with or without diffuser), a hair straightener, a hair washer, a hair cap, a hair helmet, a hair sprayer;
- provides that the step of applying ozone to the hair (b) takes place when the hair is damp. Without being bound by any theory, the Applicant submits that, in the presence of moisture or a certain percentage of water on or near the hair, the contact and adherence of ozone with the hair proteins is facilitated, thereby increasing its repairing efficacy. According to the Applicant, ozone is, in fact, able to favour the closure of the hair cuticles, increasing water retention in the hair. This increases the humidity of the hair and makes it more elastic, achieving good results in terms of hair hydration and tensile strength. In addition, the presence of water reduces the concentration of ozone in the air resulting in a less aggressive mixture, but still capable of oxidising the free sulphydryl groups SH and restoring the S-S disulphide bonds of the hair proteins;
- gives good results in terms of hydration, elasticity, and resistance to pulling/breakage.

The cosmetic method of the invention provides an economic advantage over treatment with molecular oxygen ($O_2$). Indeed, on the market, the price of oxygen concentrators is higher than that of ozone generators. The former require filters and compressors, e.g. zeolite filters, which are known to be very expensive; on the other hand, for ozone treatment, it is sufficient to have at least one ozone generator. The economic advantage reflects an advantage in terms of simplicity and ease of use of the above instrument.

## DESCRIPTION OF THE FIGURES

Figure 1 - Development of the keratin degradation parameter following ozone treatment (300 minutes).

Figure 2 - Development of the keratin degradation parameter following ozone treatment (30 minutes).

## DETAILED DESCRIPTION OF THE INVENTION

As explained above, ozone $O_3$ has a better oxidising power than oxygen $O_2$. Without wishing to be bound by any theory, the Applicant considers that, rather than a direct action of ozone, the oxidising capacity of $O_3$ can be attributed to the release of atomic oxygen O, one of the species resulting from the decomposition of ozone. In particular, atomic oxygen O is obtained by decomposing ozone, a highly unstable molecule, according to the following reaction:

$$O_3 + hv \rightarrow O_2 + O$$

According to the Applicant, ozone promotes the formation of disulphide bridges, preferably cysteine disulphide bridges, between keratin molecules, so as to restructure the hair and make it more resistant, hydrated and shiny; moreover, ozone is believed to improve the duration of technical treatments (bleaching, perming, etc.).

For the purposes of the present invention, hair care means any process or treatment intended to restore or reconstruct, in whole or in part, the natural or primordial structure of the hair or otherwise that with which the hair was characterised prior to the technical treatment.

It should be noted that the cosmetic method according to the ozone-based invention is not a trichological treatment, where trichological treatment means a treatment aimed at treating a condition, preferably afflicting the scalp. Examples of trichological treatment are: trichological hair loss treatment (hair that is fine, damaged and/or has a tendency to fall out and break), anti-dandruff treatment or antiseborrhoeic treatment.

Wet hair, for the purposes of the present invention, is understood to mean both hair that has been wet or freshly washed or immersed in water, i.e., that has not been towel-dried, wrung or rubbed; and hair that has been towel-dried, wrung or rubbed after wetting or washing.

### (a) Conducting a technical hair treatment

As previously mentioned, the cosmetic method of hair care comprises the phase of conducting a technical treatment on the hair (a), in turn comprising at least the sub-phase of:

a1) apply to the hair one or more compositions functional to the technical treatment
a2) leave one or more compositions functional to the technical treatment on the hair
(a3) wash the hair to remove one or more of the compositions functional to the technical treatment from the hair.

According to the cosmetic method of the invention, the technical treatment is chosen from among: hair reconstruction, hair conditioning, colouring, bleaching, perming and combinations of the above.

For the purposes of the invention, the natural shape or structure of the hair is determined by the arrangement of the peptide chains forming the hair, preferably bonds between cysteines, and the cross-links holding them together. These bonds are preferably chosen from the group consisting of: peptide, disulphide, salt, hydrogen bridges, and combinations of the foregoing.

Hair cleansing refers to a technical treatment aimed at washing the hair. Preferably, cleansing may take place with products that induce a modification of the natural structure of the hair, e.g. shampoo-type products, e.g. shampoos based on surfactants, e.g. relatively aggressive surfactants, and/or keratin.

Hair reconstruction is defined as a technical treatment aimed at modifying or restoring the natural structure of the hair.

Hair conditioning refers to a technical treatment with the main purpose of detangling the hair, preferably after washing or cleansing with shampoo. Conditioning products contain surfactants that create a film that envelops the hair, making it slippery and easier to comb.

Colouring refers to a technical treatment by which the natural colour of the hair is modified. Colouring includes dyes of natural or synthetic origin. Colouring treatments preferably include: temporary colouring; semi-permanent colouring; toning; permanent colouring.

Bleaching is defined as any technical treatment able to lighten/bleach the hair by a few tones, by means of a chemical process (destructive oxidation of melanin). Bleached hair usually undergoes an aggressive treatment that changes its structure, making it thinner and drier.

Discolouration treatments preferably include: lightening treatments, streaks, shatush, balayage, degradé.

Streaks is a technical bleaching treatment to lighten the hair. It is suitable for those with light-coloured hair, natural or otherwise. It involves bleaching thin, fairly regular strands along the entire length.

Shatush is a technical treatment involving lightly backcombing the locks and then bleaching the escaped locks: more intense at the tips, barely noticeable near the roots. In addition, unlike streaks, shatush cannot be resumed, i.e. the bleached lengths must be cut off in order to be able to do it a second time.

Balayage refers to a technical treatment involving the use of bleaching agents and illuminants to achieve an evenly shaded effect throughout the hair.

Degrade is a technical lightening treatment, carried out vertically, obtaining a blended effect whereby the lightening is accentuated on the lengths and tips.

According to a preferred form of the cosmetic method of the invention, the technical treatment is chosen between bleaching and perming.

"Functional compositions for technical treatment" means compositions/formulations/products based on natural/semi-natural/synthetic chemicals used or applied to hair for any type of technical treatment. For example, for technical hair cleansing treatment, functional compositions may be: shampoos, preferably based on relatively aggressive surfactants and/or keratin, conditioners; for the technical hair reconstruction treatment, functional compositions may be shampoos, nourishing masks, regenerating and conditioning lotions/mousses/sprays, preferably for dry, brittle and damaged hair; for the technical hair conditioning treatment, functional compositions are conditioners, conditioners, balms, masks, mineral salts; for the technical treatment of colouring, functional compositions are permanent colours, gradual or metallic colours, temporary colours, semi-permanent colours, vegetable colours, toning agents, highlights; for the technical treatment of bleaching, functional compositions are bleaching/lightening formulations, preferably based on oxidising agents, such as hydrogen peroxide, and/or based on ammonia; for the technical treatment of perming, functional compositions are formulations with remodelling/waving or styling capabilities of the hair structure and/or neutralising formulations (to avoid excessive structural damage to the hair).

It should be noted that the step of washing the hair to remove the functional composition(s) from the hair involves removing or permanently eliminating all traces of said functional composition(s) from the technical treatment. Preferably, said washing step is conducted with water.

According to a preferred form of implementation, the phase of conducting a technical treatment on the hair (a) may comprise further sub-phases $(a_n)$ which may be prior, subsequent or intermediate to the phases (a1)-(a3) of application, laying and rinsing of the functional composition for the technical treatment. Said further sub-phases $(a_n)$ are chosen from the group consisting of:

- apply curlers or hairpins or other suitable tools for shaping or styling hair to the hair,
- apply a further functional composition to the hair, preferably without rinsing, i.e. without washing with water to moving away/remove the composition from the hair;
- apply a further functional composition to the hair, preferably with rinsing, i.e. washing with water to moving away/remove the composition from the hair,
- rub or wring out or dab the hair, possibly with the help of a towel

and combinations of the above.

*The technical treatment is bleaching*

According to a preferred form of implementation, the technical treatment is bleaching and the phase of conducting a technical treatment on the hair (a) preferably comprises at least the sub-phases of:

- apply to the hair a first functional composition for the technical treatment, said functional composition being a bleaching composition,
- leave the bleaching composition in place on the hair,
- wash the hair to remove the bleaching composition from the hair.

Preferably, the technical treatment is bleaching and the phase of conducting a technical treatment on the hair (a) comprises the sub-phases of:

a1') apply a first functional composition to the hair, preferably a bleaching or lightening composition,

a2') leave the first functional treatment composition on the hair, preferably for a tine comprised between 40 and 50 minutes,

a3') wash the hair to remove the first functional composition of the technical treatment from the hair.

According to an optional form of realisation, bleaching comprises further sub-phases following the hair washing phase:

a4') apply to the hair a second composition functional to the technical treatment, this composition being preferably a toning composition,

a5') leave the second technical treatment composition on the hair, preferably for a time comprised between 15 and 30 minutes,

a6') wash the hair with water to remove the second functional composition of the technical treatment from the hair.

A toning composition is a chemical colouring composition; unlike permanent colours, which act deeper into the hair structure, a toning composition requires the coloured pigment to remain only on the surface of the hair. Toning compositions differ according to their composition and colouring intensity. Toning compositions can often dry out the hair due to the presence of alcohol in them.

Still preferably, following the hair washing sub-phase and before the ozone application phase (b), bleaching includes a further sub-phase of dabbing, wringing or rubbing the damp hair, possibly with the help of a towel.

Preferably, at the conclusion of the bleaching treatment, preferably following the sub-phase of washing the hair or dabbing, wringing or rubbing damp hair, the cosmetic method of the invention comprises the phase of applying ozone to damp hair (b).

*The technical treatment is permanent*

According to a preferred alternative form of implementation, the technical treatment is perming and the phase of conducting a technical treatment on the hair (a) preferably includes at least the sub-phases of:

- select one or more strands of hair and roll them up into curlers,
- apply to the hair a first functional composition for the technical treatment, said first functional composition being a waving composition,
- leave the waving composition in place on the hair,
- wash the hair to remove the wavy composition,
- apply to the hair a second functional composition to the technical treatment, said second functional composition being a neutralising composition,
- leave the neutralising composition on the hair,
- remove the curlers and wash the hair to remove the neutralising composition.

Preferably, the technical treatment is perming, and the phase of conducting a technical treatment on hair (a) comprises the sub-phases of

a0") select one or more strands of hair and roll them in curlers,

a1") apply to the coiled hair a first functional composition to the technical treatment, said first functional composition being preferably a hair shape modelling product (or waving composition),

a2") leave the first technical treatment composition on the hair, preferably for a time comprised between 5 and 30 minutes,

a3") wash the hair to remove the first functional composition of the technical treatment from the hair.

Still preferably, the technical treatment of the perm includes the following further sub-phases:

a4") apply a second functional composition to the hair, said second functional composition being preferably a neutralising composition,

a5") leave the second technical treatment composition on the hair,

(a6') remove curlers or any other instrument equivalent to them from the hair,

a7") wash the hair to remove the second, optionally the third, technical treatment functional composition from the hair.

Still preferably, following the hair washing sub-phase and before the ozone application phase (b), the perm comprises a further phase of dabbing, wringing or rubbing the damp hair.

Preferably, at the conclusion of the perming treatment, preferably following the sub-step of washing the hair or dabbing, wringing or rubbing the hair, the cosmetic method of the invention comprises the step of applying ozone to damp hair (b).

### (b) Step of applying ozone to hair

Preferably, the step of applying ozone to the hair (b) involves ozone in gaseous form, mixed with water or in the form of humidified ozone (ozonised water).

For the purposes of this invention, the expression "applying ozone to hair" (b) shall preferably mean

- dispense gaseous ozone directly onto the hair; alternatively,

- apply a mixture of ozone and water to the hair; alternatively

- apply water-soluble ozone (humidified ozone or ozonised water) to the hair.

For example, ozone in gaseous form can preferably be generated from an ozone generator device. Preferably, an ozone generator comprises at least one fan and two plates, one of which is powered to generate electrical discharges; oxygen conveyed by the fan passes between the plates. Preferably, the oxygen carried between the two plates by the fan is oxygen present in the atmosphere.

Preferably, the oxygen conveyed between the two plates is pure oxygen, i.e. with a purity of $\geq 98\%$, such as that contained in an $O_2$ cylinder or generated by an $O_2$ concentrator.

### Ozone application time and dosage

The phase of applying ozone to the hair (b) is conducted for or within a period of time > 0.5 and < 21 minutes, preferably $\geq 1$ and < 21 minutes, preferably $\geq 1.5$ and < 21 minutes, preferably $\geq 2$ and < 21 minutes, preferably $\geq 3$ and < 21 minutes, preferably > 3 and $\leq 20$ minutes, preferably > 3 and < 15 minutes, preferably > 3 and $\leq 10$ minutes, preferably $\geq 5$ and $\leq 10$ minutes, preferably $\geq 6$ and $\leq 10$ minutes, preferably is 10 minutes.

Excluding the ozone dosage range of 180 to 400 mg/h, the ozone dosage range is comprised between $\geq 0.01$ and $\leq 2000$ mg/h, preferably $\geq 0.05$ and $\leq 2000$ mg/h, preferably $\geq 0.1$ and $\leq 2000$ mg/h, preferably $\geq 0.5$ and $\leq 2000$ mg/h, preferably $\geq 1$ and $\leq 2000$ mg/h, preferably $\geq 2$ and $\leq 2000$ mg/h, preferably > 2.7 and $\leq 2000$ mg/h, preferably > 3 and $\leq 2000$ mg/h, preferably $\geq 5$ and $\leq 2000$ mg/h, preferably $\geq 10$ and $\leq 2000$ mg/h, preferably $\geq 15$ and $\leq 2000$ mg/h, preferably $\geq 20$ and $\leq 2000$ mg/h, preferably $\geq 23$ and $\leq 2000$ mg/h, preferably $\geq 25$ and $\leq 2000$ mg/h, preferably $\geq 27$ and $\leq 2000$ mg/h, preferably $\geq 30$ and $\leq 2000$ mg/h, preferably $\geq 40$ and $\leq 2000$ mg/h, preferably $\geq 50$ and $\leq 2000$ mg/h, preferably $\geq 60$ and $\leq 2000$ mg/h, preferably $\geq 70$ and $\leq 2000$ mg/h, preferably $\geq 80$ and $\leq 2000$ mg/h, preferably $\geq 90$ and $\leq 2000$ mg/h, preferably $\geq 100$ and $\leq 2000$ mg/h, preferably $\geq 120$ and $\leq 2000$ mg/h, preferably $\geq 130$ and $\leq 2000$ mg/h, preferably $\geq 140$ and $\leq 2000$ mg/h, preferably $\geq 150$ and $\leq 2000$ mg/h, preferably $\geq 160$ and $\leq 2000$ mg/h, preferably $\geq 170$ and < 2000 mg/h, preferably $\geq 410$ and < 2000 mg/h preferably $\geq 420$ and $\leq 2000$ mg/h, preferably $\geq 430$ and $\leq 2000$ mg/h, preferably $\geq 450$ and $\leq 2000$ mg/h, preferably $\geq 500$ and $\leq 2000$ mg/h, preferably $\geq 600$ and $\leq 2000$ mg/h, preferably $\geq 630$ and $\leq 2000$ mg/h, preferably $\geq 650$ and $\leq 2000$ mg/h, preferably $\geq 670$ and $\leq 2000$ mg/h, preferably $\geq 680$ and $\leq 2000$ mg/h, preferably $\geq 700$ and $\leq 2000$ mg/h, preferably $\geq 800$ and $\leq 2000$ mg/h preferably $\geq 900$ and $\leq 2000$ mg/h, preferably $\geq 1000$ and $\leq 2000$ mg/h, preferably $\geq 1100$ and $\leq 2000$ mg/h, preferably $\geq 1200$ and $\leq 2000$ mg/h, preferably $\geq 1300$ and $\leq 2000$ mg/h, preferably $\geq 1400$ and $\leq 2000$ mg/h, preferably $\geq 1500$ and $\leq 2000$ mg/h, preferably $\geq 1600$ and $\leq 2000$ mg/h, preferably $\geq 1700$ and $\leq 2000$ mg/h, preferably $\geq 1800$ and $\leq 2000$ mg/h, preferably $\geq 1900$ and $\leq 2000$ mg/h

According to a preferred form, excluding the ozone dosage range of 180 to 400 mg/h, preferably the ozone dosage range is comprised between $\geq 0.01$ and $\leq 170$ mg/h and between $\geq 410$ and $\leq 2000$ mg/h, preferably the ozone dosage range is comprised between ≥ 0,01 and ≤ 170 mg/h and between ≥ 650 and ≤ 2000 mg/h, preferably ozone dosage is between ≥ 0,01 and ≤ 170 mg/h and between ≥ 700 and ≤ 2000 mg/h, preferably ozone dosage is between ≥ 0,01 and ≤ 150 mg/h and between ≥ 700 and ≤ 2000 mg/h.

The Applicant specifies that, as long as one is within the time and dosage parameters of ozone according to the ozone application step (b) of the present invention, the restructuring effect of ozone on the hair is achieved. Thus, for the purposes of the invention, by following the time and dosage intervals of ozone application, it is possible to achieve a restructuring or reconstruction effect of the primordial structure of the hair, i.e., that which has been treated prior to the technical treatment. On the other hand, longer ozone application times risk damaging the hair, with deconstructing effects comparable to those resulting from certain technical treatments, such as bleaching or perming.

At a dosage > 2000 mg/h, there is a reduction in the effectiveness and/or safety of ozone use, e.g. temporary or permanent structural damage to the hair could be induced.

Preferably within a time period preferably > 0.5 and < 21 minutes, the phase of applying ozone to the hair (b) requires ozone in an amount preferably comprised between 9 mg and 700 mg, preferably between 9 mg and 500 mg, preferably between 10 and 300 mg, preferably between 10 and 200 mg, preferably between 10 and 100 mg, preferably between 10 and 80 mg, preferably 70 mg.

Excluding the range between 0.0014 L/min and 0.0031 L/min, the step of applying ozone to the hair (b) preferably provides for an ozone delivery flow comprised between 0.001 and 10 L/min, preferably between 0.002 and 10 L/min, preferably between 0.003 and 10 L/min, preferably between 0.005 and 10 L/min, preferably between 0.005 and 8 L/min, preferably between 0.005 and 7 L/min, preferably between 0.005 and 5 L/min, preferably between 0.005 and 3 L/min, preferably between 0.005 and 1 L/min, preferably between 0.01 and 5 L/min, preferably between 0.1 and 5 L/min, preferably between 0.5 and 5 L/min. Still preferably, except for the range of 0.0014 L/min to 0.0031 L/min, the step of applying ozone to the hair (b) comprises an ozone delivery flow rate of between 0.5 and 10 L/min, preferably comprised between 1 and 10 L/min, preferably between 3 and 10 L/min, preferably between 5 and 10 L/min, preferably between 7 and 10 L/min.

The delivery pressure of ozone in gaseous form is preferably atmospheric.

### Ozone application step (b) on damp hair

For the purposes of this finding, the ozone application step (b) is conducted on essentially damp hair. Moist hair is defined as

- the hair is towel-dried, optionally rubbed or wrung out, using a cloth or towel, after wetting the hair or after washing, in order to remove excess water, thus preventing the hair from dripping when excessively wet; or
- the hair wet, i.e. after washing or just after wetting the hair, partially or totally; or
- the hair immersed in water.

### Ozone application phase (b) at the conclusion of a technical hair treatment (a)

The step of applying ozone to wet hair (b) takes place at the *conclusion* of the step of conducting a technical treatment on the hair (a), i.e. after the last sub-step constituting the technical treatment (a); according to a preferred embodiment, the step of applying ozone (b) is conducted on the wet hair after rinsing or washing or cleansing thoroughly with water to remove all traces of the composition(s) functional to the technical treatment.

According to the Applicant, this allows good results to be achieved in terms of hair care, since the phase of applying ozone to the hair (b) takes place at the *conclusion* of the technical treatment, in other words *following* or *subsequent to* the completed technical treatment; without wishing to be bound by any theory, the Applicant considers that the use of ozone at the conclusion of the technical treatment allows better restructuring and regeneration of the hair.

### Ozone application handpieces

The step of applying ozone to the hair (b) is preferably done with a handpiece chosen from the group consisting of: a hairbrush, a hair comb, a hair dryer - preferably with or without a diffuser - a hair straightener, a hair washer, a hair cap, a hair helmet, a hair sprayer.

### First mode of ozone application

According to a preferred form of implementation, the phase of applying ozone to the hair (b) preferably comprises the following sub-phases:

(b1) prepare ozone in gaseous form;

(b2) prepare water in liquid form;

b3) dispense ozone in gaseous form and, at the same time, nebulize water in liquid form on the hair.

For the purposes of the present invention, the nebulisation of water is preferably carried out by means of a nebuliser, i.e. a device that transforms a liquid into an aerosol of very fine droplets; the liquid, injected by a nozzle, is dispersed by a diffuser.

The phase of dispensing ozone in gaseous form and, at the same time, spraying water in liquid form on the hair (b3) preferably involves dispensing ozone in gaseous form on the hair and, at the same time, spraying liquid water on the damp hair.

Preferably, the step of applying ozone to the hair (b) according to this mode is done by dispensing ozone in gaseous form at a dosage comprised between 50 and 1000 mg/h, preferably between 50 and 800 mg/h, preferably between 50 and 500 mg/h, preferably between 50 and 170 mg/h, preferably between 50 and 160 mg/h, preferably between 50 and 150 mg/h, preferably between 60 and 130 mg/h, preferably between 70 and 100 mg/h, excluding the interval comprised between 180 and 400 mg/h.

*Second method of ozone application*

In a second alternative form of implementation to the previous one, the step of applying ozone to the hair (b) preferably comprises the following sub-steps:

b1') prepare ozone in gaseous form;

b2') dispense ozone in gaseous form on damp hair.

It should be noted that, according to this second form of realisation and unlike the first method of applying ozone, no water in nebulised liquid form is used. Instead, the moisture already present on the damp hair is exploited.

Preferably, the step of applying or dispensing ozone on the hair (b) according to this modality is done by dispensing ozone in gaseous form at a dosage between 410 mg and 1500 mg in one hour (h) (extremes included), preferably between 450 and 1400 mg/h, preferably between 500 and 1300 mg/h, preferably between 800 and 1200 mg/h, excluding the range between 180 and 400 mg/h.

*Third mode of ozone application*

A third embodiment of the invention provides that the step of applying ozone to hair (b) preferably comprises the following sub-steps:

b1") prepare ozone in gaseous form

b2") prepare water in liquid form,

b3") solubilise ozone in gaseous form in water to obtain humidified ozone (or ozonated water), and

b4") apply humidified ozone to damp hair.

Preferably, the step of solubilising the ozone in gaseous form in water to obtain humidified ozone (or ozonised water) (b3") involves the use of a bubbler device, known to the practitioner, for solubilising the ozone in gaseous form in water. In other words, the gaseous ozone is humidified, by means of a bubbler, before being applied to the hair.

A further way of carrying out the ozone solubilisation phase in gaseous form in water to obtain humidified ozone (or ozone humidification phase) (b3") preferably involves mixing or solubilising ozone in gaseous form directly in water, preferably the water from a sink or wash basin. In this way, gaseous ozone is "immersed" (solubilised) in water before being applied to the hair.

Preferably, the passage of the ozone stream through water allows the gas to dissolve, so that the microdroplets of humidified ozone (or ozonised water) adhere more effectively to the hair, improving its efficacy. For the purposes of the present invention, preferably the bubbler has a volume $\geq 350$ ml and $\leq 500$ ml, preferably equal to 500 ml.

For the purposes of this finding, "applying humidified ozone to wet hair" (b4") means applying humidified or water-solubilised ozone (humidified ozone or ozonised water) to wet hair.

Preferably, the step of applying humidified ozone (or ozonised water) to the hair (b4") takes place by nebulisation ("vaporisation") of the humidified ozone, i.e. by means of a nebuliser or vaporiser; according to another embodiment, the step of applying humidified ozone to the hair (b4") takes place preferably by pouring (or outflowing) at least a part of the aforementioned humidified ozone (or ozonised water) onto the already wet hair of a user.

In an alternative form of implementation, the method may involve the use of a catalyst that helps to improve the effectiveness of the treatment by recovering chemical species produced during ozone application that would be lost to the environment if not recovered.

Preferably, the step of applying or dispensing ozone on the hair (b) according to the third modality takes place preferably by applying ozone in gaseous form at a dosage between 410 mg and 1500 mg in one hour (h) (extremes included), preferably between 450 and 1400 mg/h, preferably between 500 and 1300 mg/h, preferably between 800 and 1200 mg/h, excluding the range between 180 and 400 mg/h.

According to the third ozone application method, considering the solubility of ozone in gaseous form in water at a temperature preferably between 20 and 30°C, the ozone titre (or concentration) in humidified ozone (or ozonised water) is preferably between 350 mg and 600 mg, preferably between 400 mg and 570 mg per litre of humidified ozone (or ozonised water).

Preferably, following the step of applying ozone to the hair (b), respecting the timing for the purposes of the invention, the hair may be dried, preferably by means of an ordinary hair dryer.

## EXAMPLES

Below, the Applicant gives examples of the invention for illustrative and non-limiting purposes only.

### Example 1 - Study of the effect of ozone treatment on standard bleached hair

This study uses an ozone generator at 1000 mg/h. The study was conducted by treating standard bleached hair with ozonised air (containing electrophilic oxygen), using an ozone generator at 1000 mg/h, monitoring the ozone concentration, temperature and humidity of the treatment environment.

Ozone-containing air was used either directly ("$O_3$ direct"), or after being passed through a bubbler ("$O_3$ bubbled") into water. The operating conditions are listed in table 1 below:

Table 1

| Experiment | Temperature | Relative Humidity |
|---|---|---|
| 1000 mg/h $O_3$ direct | 18-20 °C | 52-60 % |
| 1000 mg/h $O_3$ bubbled | 18-20 °C | 70-75 % |

The final state of the hair was determined by measuring the degradation of cysteine by monitoring the ratio of the areas of the FT-IR signals related to cysteic acid (1240 cm$^{-1}$), which represents the degraded cysteine, to the chain amide signal (1630 - 1640 cm$^{-1}$), which represents the total amount of protein. Table 2 below shows the keratin degradation index value as a function of time over 300 minutes of electrophilic oxygen treatment.

Table 2

| Time (minutes) | 1000 mg/h $O_3$ direct | 1000 mg/h $O_3$ bubbled |
|---|---|---|
| 0 | 0,34 | 0,34 |
| 2 | 0,16 | 0,20 |
| 5 | 0,15 | 0,18 |
| 7 | 0,15 | 0,16 |
| 10 | 0,20 | 0,19 |
| 15 | 0,25 | 0,18 |
| 20 | 0,27 | 0,19 |
| 30 | 0,32 | 0,25 |
| 60 | 0,35 | 0,28 |
| 120 | 0,37 | 0,32 |
| 180 | 0,40 | 0,35 |
| 240 | 0,41 | 0,36 |
| 300 | 0,40 | 0,36 |

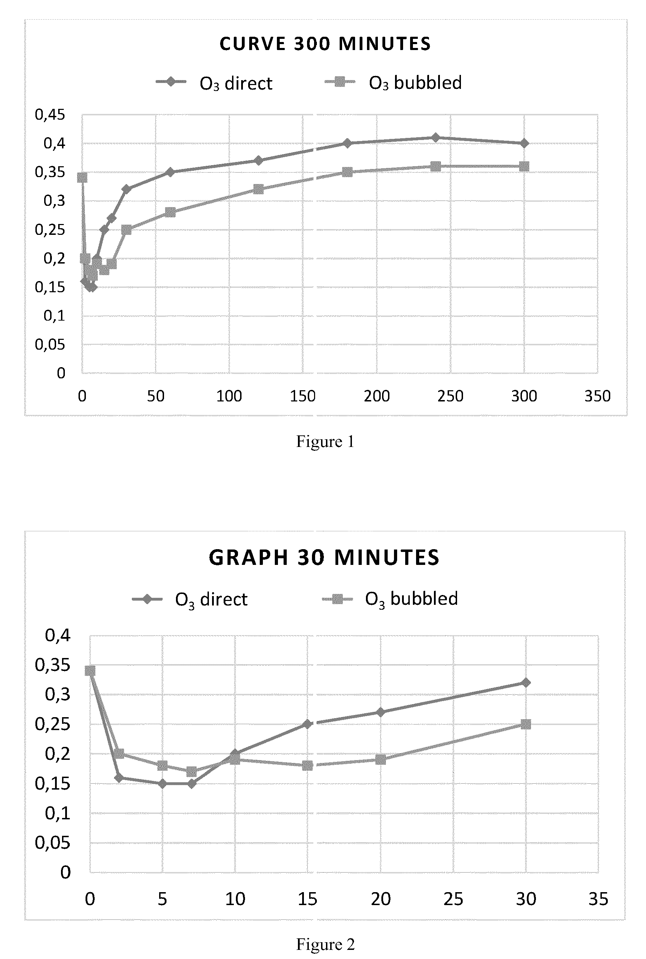

Figures 1 and 2 show the same data in graphical format, highlighting the trend up to 300 minutes and that in the first 30 minutes.

***Example 2 - Optimisation of parameters for ozone treatment***

The aim of the study is to optimise the parameters for ozone treatment on bleached hair. Samples of 10 g of standard hair decolourised for 15 minutes with 30-volume hydrogen peroxide (9% in weight) and subsequently treated with ozone at different flow rates and times (always remaining within the claimed time and dosage intervals of ozone) were used. The controlled parameter is the IR degradation index of keratin, calculated from the infrared spectrum signals related to keratin degradation at 1041 cm$^{-1}$. The following equation (1) was developed to calculate the optimal treatment time:

$$Optimum\ time = 21 - 6 \cdot F$$

where F is the flow measured in L/min; time is measured in minutes.

It is possible to calculate a percentage hair regeneration index (R), understood as the ratio of the difference between the initial (I(initial)) and the current (I(current)) degradation index, divided by the initial (I(initial)) per cent, as equation (2) below:

$$R = \frac{I(initial) - I(current)}{I(initial)} \cdot 100$$

For this hair sample I(initial) is 0.45 and is reduced to a maximum of 0.15.

By mathematically processing the trend, we obtain the following empirical equation (3), valid in the range 0-20 minutes:

$$R = \frac{67 \cdot t}{21 - 6 \cdot F}\left(2 - \frac{t}{21 - 6 \cdot F}\right)$$

**Claims**

1. Cosmetic method for hair care comprising the following steps:

    a) conducing a technical treatment on the hair, comprising at least the sub-steps of

        a1) applying on the hair one or more compositions functional to the technical treatment,
        a2) leaving one or more compositions functional to the technical treatment in place on the hair,
        a3) washing the hair to remove from the hair the one or more compositions functional to the technical treatment,

    b) applying ozone on damp hair

        for a period of time > 0,5 and < 21 minutes,
        at a ozone dosage comprised between ≥ 0.01 and ≤ 2000 mg/h, excluding the ozone dosage range comprised between 180 and 400 mg/h,

    wherein

        the phase of applying ozone to damp hair (b) takes place at the end of the phase of conducting a technical treatment on the hair (a),
        the technical treatment is selected from: hair reconstruction, hair conditioning, colouring, bleaching, perming, and combinations of the foregoing.

2. Cosmetic method according to claim 1, wherein the ozone dosage is ≥ 0.05 mg/h and ≤ 2000 mg/h.

3. Cosmetic method according to any one of claims from 1 to 2, wherein the technical treatment is selected from bleaching and perming.

4. Cosmetic method according to any one of claims from 1 to 3, wherein the step of applying the ozone on the hair (b) comprises the following sub-steps:

   b1) arranging ozone in gaseous form;
   b2) arranging water in liquid form;
   b3) dispensing ozone in gaseous form on the hair and, at the same time, nebulizing the water in liquid form on the damp hair;
   **or**
   b1') arranging ozone in gaseous form;
   b2') dispensing ozone in gaseous form on the damp hair.

5. Cosmetic method according to any one of claims from 1 to 4, wherein the step of applying the ozone on the hair (b) comprises the following sub-steps:

   b1") arranging ozone in gaseous form
   b2'') arranging water in liquid form,
   b3") solubilizing the ozone in gaseous form in the water to obtain humidified ozone, and
   b4") applying the humidified ozone on the damp hair.

6. Cosmetic method according to claim 5, wherein the ozone content in the humidified ozone is comprised between 350 mg and 600 mg per litre of humidified ozone.

7. Cosmetic method according to any one of claims from 1 to 6, wherein the step of applying the ozone on the hair (b) occurs by a means selected from the group consisting of: a hair brush, a hair comb, a hair dryer (with or without diffuser), a hair straightener, a headwash, a hair cap, a hooded dryer, a nebulizer.

8. Cosmetic method according to any one of claims from 1 to 7, wherein the step of applying ozone on the hair (b) includes a dispensing flow comprised between 0.001 and 10 L/min.


**Patentansprüche**

1. Kosmetisches Haarpflegeverfahren, umfassend die folgenden Schritte: a) Durchführen einer technischen Behandlung am Haar, umfassend mindestens die Teilschritte: a1) Aufbringen einer oder mehrerer für die technische Behandlung funktioneller Zusammensetzungen auf das Haar, a2) Einwirkenlassen der einen oder der mehreren für die technische Behandlung funktionellen Zusammensetzungen auf dem Haar, a3) Waschen des Haares, um die eine oder die mehreren für die technische Behandlung funktionellen Zusammensetzungen aus dem Haar zu entfernen, b) Aufbringen von Ozon auf feuchtes Haar für eine Zeitdauer von > 0,5 und < 21 Minuten, bei einer Ozondosis von $\geq 0,01$ bis $\leq 2000$ mg/h, ausschließlich des Ozondosisbereichs von 180 bis 400 mg/h, wobei die Phase des Aufbringens von Ozon auf feuchtes Haar (b) am Ende der Phase der Durchführung einer technischen Behandlung am Haar (a) stattfindet, die technische Behandlung ausgewählt ist aus: Haarwiederaufbau, Haarkonditionierung, Färben, Bleichen, Dauerwellbehandlung und Kombinationen davon.

2. Kosmetisches Verfahren nach Anspruch 1, wobei die Ozondosis $\geq 0,05$ mg/h und $\leq 2000$ mg/h beträgt.

3. Kosmetisches Verfahren nach einem der Ansprüche 1 bis 2, wobei die technische Behandlung aus Bleichen und Dauerwellbehandlung ausgewählt ist.

4. Kosmetisches Verfahren nach einem der Ansprüche 1 bis 3, wobei der Schritt des Aufbringens des Ozons auf das Haar (b) die folgenden Teilschritte umfasst: b1) Bereitstellen von Ozon in gasförmiger Form; b2) Bereitstellen von Wasser in flüssiger Form; b3) Abgeben von Ozon in gasförmiger Form auf das Haar und gleichzeitiges Vernebeln des Wassers in flüssiger Form auf das feuchte Haar; oder b1') Bereitstellen von Ozon in gasförmiger Form; b2') Abgeben von Ozon in gasförmiger Form auf das feuchte Haar.

5. Kosmetisches Verfahren nach einem der Ansprüche 1 bis 4, wobei der Schritt des Aufbringens des Ozons auf das Haar (b) die folgenden Teilschritte umfasst: b1") Bereitstellen von Ozon in gasförmiger Form, b2") Bereitstellen von Wasser in flüssiger Form, b3") Lösen des Ozons in gasförmiger Form in dem Wasser, um befeuchtetes Ozon zu erhalten, und b4") Aufbringen des befeuchteten Ozons auf das feuchte Haar.

6. Kosmetisches Verfahren nach Anspruch 5, wobei der Ozongehalt in dem befeuchteten Ozon zwischen 350 mg und 600 mg pro Liter befeuchteten Ozons liegt.

7. Kosmetisches Verfahren nach einem der Ansprüche 1 bis 6, wobei der Schritt des Aufbringens des Ozons auf das Haar (b) durch ein Mittel erfolgt, das aus der Gruppe ausgewählt ist, bestehend aus: einer Haarbürste, einem Haarkamm, einem Haartrockner (mit oder ohne Diffusor), einem Haarglätter, einem Haarwaschbecken, einer Haarhaube, einer Trockenhaube, einem Vernebler.

8. Kosmetisches Verfahren nach einem der Ansprüche 1 bis 7, wobei der Schritt des Aufbringens von Ozon auf das Haar (b) einen Abgabedurchfluss von 0,001 bis 10 L/min einschließt.

## Revendications

1. Procédé cosmétique de soin capillaire comprenant les étapes suivantes : a) la réalisation d'un traitement technique sur les cheveux, comprenant au moins les sous-étapes consistant à : a1) appliquer sur les cheveux une ou plusieurs compositions fonctionnelles pour le traitement technique, a2) laisser en place sur les cheveux une ou plusieurs compositions fonctionnelles pour le traitement technique, a3) laver les cheveux pour éliminer des cheveux la ou les compositions fonctionnelles pour le traitement technique, b) l'application d'ozone sur des cheveux humides pendant une durée > 0,5 et < 21 minutes, à un dosage d'ozone compris entre ≥ 0,01 et ≤ 2000 mg/h, à l'exclusion de la plage de dosage d'ozone comprise entre 180 et 400 mg/h, dans lequel la phase d'application d'ozone sur des cheveux humides (b) a lieu à la fin de la phase de réalisation d'un traitement technique sur les cheveux (a), le traitement technique est choisi parmi : la reconstruction capillaire, le conditionnement capillaire, la coloration, la décoloration, la permanente, et des combinaisons de ce qui précède.

2. Procédé cosmétique selon la revendication 1, dans lequel le dosage d'ozone est ≥ 0,05 mg/h et ≤ 2000 mg/h.

3. Procédé cosmétique selon l'une quelconque des revendications 1 à 2, dans lequel le traitement technique est choisi parmi la décoloration et la permanente.

4. Procédé cosmétique selon l'une quelconque des revendications 1 à 3, dans lequel l'étape d'application de l'ozone sur les cheveux (b) comprend les sous-étapes suivantes : b1) préparer de l'ozone sous forme gazeuse ; b2) préparer de l'eau sous forme liquide ; b3) dispenser de l'ozone sous forme gazeuse sur les cheveux et, simultanément, nébuliser l'eau sous forme liquide sur les cheveux humides ; ou b1') préparer de l'ozone sous forme gazeuse ; b2') dispenser de l'ozone sous forme gazeuse sur les cheveux humides.

5. Procédé cosmétique selon l'une quelconque des revendications 1 à 4, dans lequel l'étape d'application de l'ozone sur les cheveux (b) comprend les sous-étapes suivantes : b1") préparer de l'ozone sous forme gazeuse b2") préparer de l'eau sous forme liquide, b3") solubiliser l'ozone sous forme gazeuse dans l'eau pour obtenir de l'ozone humidifié, et b4") appliquer l'ozone humidifié sur les cheveux humides.

6. Procédé cosmétique selon la revendication 5, dans lequel la teneur en ozone dans l'ozone humidifié est comprise entre 350 mg et 600 mg par litre d'ozone humidifié.

7. Procédé cosmétique selon l'une quelconque des revendications 1 à 6, dans lequel l'étape d'application de l'ozone sur les cheveux (b) s'effectue au moyen d'un dispositif choisi dans le groupe constitué de : une brosse à cheveux, un peigne, un sèche-cheveux (avec ou sans diffuseur), un fer à lisser, un lave-tête, un bonnet pour cheveux, un casque séchant, un nébuliseur.

8. Procédé cosmétique selon l'une quelconque des revendications 1 à 7, dans lequel l'étape d'application de l'ozone sur les cheveux (b) inclut un débit de distribution compris entre 0,001 et 10 L/min.

## CURVE 300 MINUTES

O₃ direct      O₃ bubbled

Figure 1

## GRAPH 30 MINUTES

O₃ direct      O₃ bubbled

Figure 2

## REFERENCES CITED IN THE DESCRIPTION

**Patent documents cited in the description**

- FR 3010305
- EP 0103547 A